# FASCICULE DE BREVET EUROPEEN

(11) **EP 4 140 536 B1**
(45) Date de publication et mention de la délivrance du brevet: **02.10.2024**
(21) Numéro de dépôt: 21315143.4
(22) Date de dépôt: 25.08.2021
(51) Int. Cl.: A61N 1/378, A61N 1/375, H02N 2/18, A61N 1/372, H10N 30/30

(54) **MODULE DE RÉCUPÉRATION D'ÉNERGIE À TRANSDUCTEUR PIÉZOÉLECTRIQUE À CONSTRUCTION COMPACTE, NOTAMMENT POUR L'ALIMENTATION D'UNE CAPSULE CARDIAQUE AUTONOME LEADLESS**
ENERGIERÜCKGEWINNUNGSMODUL MIT PIEZOELEKTRISCHEM WANDLER IN KOMPAKTBAUWEISE, INSBESONDERE FÜR DIE STROMVERSORGUNG EINER LEITUNGSLOSEN AUTONOMEN HERZKAPSEL
ENERGY RECOVERY MODULE WITH A COMPACT-CONSTRUCTION PIEZOELECTRIC TRANSDUCER, IN PARTICULAR FOR THE POWER SUPPLY OF A LEADLESS AUTONOMOUS CARDIAC CAPSULE

(43) Date de publication de la demande: 01.03.2023
(73) Titulaire: CAIRDAC, 92160 Antony (FR)
(72) Inventeur: REGNIER, Willy, Longjumeau 91160 (FR); NGUYEN-DINH, An, 37520 La Riche (FR); MAKDISSI, Alaa, Paris 75011 (FR); DOHIN, Julien, 92170 Vanves (FR); VASSAL, Steeven, 37200 Tours (FR); HOANG, Thien, 37000 Tours (FR)
(74) Mandataire: Dupuis-Latour, Dominique

(56) Documents cités:
- EP-A1- 3 693 056
- WO-A1-2014/116794
- US-A1- 2006 217 776
- US-A1- 2017 077 839
- US-A1- 2018 185 638

## Description

### CONTEXTE DE L'INVENTION

### Domaine de l'invention

L'invention concerne les récupérateurs d'énergie, également dénommés "harvesters" ou "scavengers", qui recueillent l'énergie mécanique résultant de mouvements divers qu'ils subissent et convertissent cette énergie mécanique en énergie électrique.

Elle concerne plus spécifiquement les récupérateurs de type dit "PEH" *(Piezoelectric Energy Harvester),* qui utilisent comme transducteur mécano-électrique une lame piézoélectrique oscillante couplée à une masse mobile inertielle.

L'invention sera décrite plus particulièrement dans une application de ces récupérateurs d'énergie à des dispositifs- médicaux autonomes, notamment les dispositifs de type capsule implantable autonome, en particulier ceux de ces dispositifs qui sont destinés à être implantés dans une cavité cardiaque.

Cette application, si elle est particulièrement avantageuse, ne doit toutefois pas être considérée comme limitative de l'invention, dont les enseignements peuvent être appliqués à de nombreux autres types de dispositifs autonomes incorporant un récupérateur d'énergie de type PEH, que ces dispositifs soient implantables ou non, qu'il s'agisse de dispositifs médicaux ou non.

### Description de la technique antérieure

Dans le domaine des implants médicaux, les récents progrès en matière de miniaturisation de dispositifs actifs et les progrès des sciences du vivant permettent dorénavant le développement d'une grande variété de systèmes miniaturisés implantables totalement autonomes, à des fins de surveillance, de diagnostic ou de traitement. Ces dispositifs mettent en oeuvre des procédures d'implantation moins invasives, plus de confort, des performances accrues et souvent ouvrent l'accès à des nouveaux types de diagnostics et traitements.

Lorsqu'elle est appliquée au domaine des implants médicaux, l'invention concerne plus précisément ceux de ces implants qui incorporent un système d'autoalimentation comprenant un récupérateur d'énergie mécanique associé à un organe de stockage d'énergie intégré tel qu'une batterie rechargeable ou une capacité à hautes performances.

En effet, l'un des aspects critiques de ces dispositifs miniaturisés est celui de l'autonomie électrique. La durée de vie d'un tel implant étant d'environ 8 à 10 ans, compte tenu des très faibles dimensions il n'est pas possible d'utiliser une batterie conventionnelle, même à forte densité.

Le récupérateur d'énergie pallie cet inconvénient en recueillant l'énergie mécanique résultant des mouvements divers subis par le corps du dispositif implanté. Ces mouvements peuvent avoir pour origine un certain nombre de phénomènes se produisant par exemple au rythme des battements cardiaques tels que les secousses périodiques de la paroi sur laquelle est ancré l'implant, les vibrations des tissus cardiaques liées entre autres aux fermetures et ouvertures des valves cardiaques, ou encore les variations de débit du flux sanguin dans le milieu environnant, qui sollicitent l'implant et le font osciller au rythme des variations de débit. L'énergie mécanique recueillie par le récupérateur est convertie en énergie électrique (tension ou courant) au moyen d'un transducteur mécanoélectrique approprié, pour assurer l'alimentation des divers circuits et capteurs du dispositif et la recharge de l'organe de stockage d'énergie. Ce système d'alimentation permet au dispositif de fonctionner en parfaite autonomie électrique pendant toute sa durée de vie.

Cette technique de récupération d'énergie est particulièrement bien adaptée à l'alimentation des capsules autonomes implantées dépourvues de toute liaison physique à un dispositif distant. Ces capsules sont dénommées pour cette raison "capsules *leadless",* pour les distinguer des électrodes ou capteurs disposés à l'extrémité distale d'une sonde (*lead*) parcourue sur toute sa longueur par un ou plusieurs conducteurs reliés à un générateur connecté à l'extrémité opposée, proximale.

L'invention n'est toutefois pas limitée à un type particulier de capsule, ni même d'implant leadless, et elle est applicable indifféremment à de nombreux autres types de dispositifs médicaux implantables, quelle que soit leur destination fonctionnelle, cardiaque ou autre, médicale ou non. Dans le cas d'une application cardiaque, la capsule leadless surveille en continu le rythme du patient et délivre si nécessaire au coeur des impulsions électriques de stimulation, de resynchronisation et/ou de défibrillation en cas de troubles du rythme détecté par la capsule. La capsule comprend par ailleurs divers circuits électroniques, capteurs, etc. ainsi que des moyens émetteurs/récepteurs de communication sans fil pour l'échange de données à distance, le tout étant intégré dans un corps de très petites dimensions qui puisse être implanté dans des sites difficilement accessibles ou laissant peu d'espace, tels que l'apex du ventricule, la paroi interne de l'oreillette, etc.

Le WO 2019/001829 A1 (Cairdac) décrit un exemple d'une telle capsule leadless intracardiaque.

Une capsule leadless intracardiaque est aussi décrit dans le EP3693056A1.

L'invention concerne plus précisément les capsules ou dispositifs implantables analogues dont le récupérateur d'énergie est du type PEH, c'est-à-dire utilisant un transducteur piézoélectrique ou "PZT" *(PieZoelectric Transducer*) et un ensemble pendulaire inertiel soumis aux sollicitations externes décrites plus haut. L'ensemble pendulaire inertiel comprend une masse mobile logée dans le corps de la capsule, dite "masse sismique" ou "masse inertielle", qui est entraînée au gré des mouvements de la capsule soumise en permanence aux diverses sollicitations externes décrites plus haut. Après chacune de ces sollicitations, la masse inertielle, qui est couplée à un élément élastiquement déformable, oscille à une fréquence propre d'oscillation libre.

L'énergie mécanique de l'oscillation est convertie en énergie électrique par un transducteur mécanoélectrique produisant un signal électrique. Ce transducteur mécanoélectrique peut en particulier être un PZT sollicité de façon cyclique en flexion de manière à engendrer au sein du matériau qui le constitue des charges électriques qui sont récupérées en surface du composant pour être utilisées par le système d'autoalimentation de la capsule leadless. Le PZT se présente le plus souvent sous la forme d'une lame encastrée à l'une de ses extrémités et couplée à la masse inertielle à son autre extrémité, qui est libre.

Le signal électrique en sortie du transducteur est délivré à un circuit de gestion de l'alimentation de la capsule, qui redresse et régule le signal électrique pour délivrer en sortie une tension ou un courant continus stabilisés permettant d'assurer l'alimentation des divers circuits électroniques et capteurs de la capsule, ainsi que la recharge de l'organe de stockage d'énergie.

Un tel récupérateur d'énergie de type PEH est notamment décrit dans le US 3,456,134 A (Ko) et dans le WO 2019/001829 A1 précité.

Un récupérateur d'énergie est aussi décrit dans le US2006217776A1.

On notera que le terme de "lame" doit être entendu dans son sens le plus large, à savoir une bande mince et plate de forme allongée, étant entendu que la forme de cette bande n'est pas nécessairement rectangulaire ni son épaisseur constante (comme dans la description du mode de réalisation particulier qui sera donnée plus bas). Le terme de "lame" au sens de la présente invention recouvre ainsi des éléments pouvant présenter une largeur et/ou une épaisseur qui ne sont pas constantes en direction longitudinale, ainsi qu'éventuellement une déformabilité pouvant aller au-delà d'un unique degré de liberté en flexion.

Le problème de l'invention réside dans la recherche d'une plus grande compacité dimensionnelle du PEH, tout en préservant ses capacités fonctionnelles de production d'énergie en quantité suffisante pour assurer l'alimentation des circuits de la capsule autonome, et la recharge de la batterie tampon intégrée.

En particulier, si l'on souhaite réaliser des capsules leadless qui puissent être implantées dans l'oreillette, la forme tubulaire allongée des capsules conventionnelles n'est pas adaptée, du fait du volume beaucoup plus réduit de la cavité atriale par rapport au ventricule, et de la nécessité en outre d'implanter la capsule sur une paroi latérale de l'oreillette : une capsule allongée devrait alors être implantée avec sa plus grande dimension plus ou moins perpendiculaire à la veine cave supérieure, ce qui concrètement n'est pas possible avec cette voie d'accès. A l'opposé, pour une capsule leadless ventriculaire implantée au fond du ventricule droit, l'apex se situe dans le prolongement de la veine cave supérieure et de la valve tricuspide, donc ne pose pas de difficulté particulière d'implantation, et de plus la forme allongée de la capsule n'est pas gênante compte tenu du plus grand volume de la cavité ventriculaire.

Il en serait de même pour une capsule épicardique, c'est-à-dire implantée à la surface extérieure du myocarde, où il serait souhaitable de disposer d'un élément de forme plutôt plate qu'allongée.

En particulier, si l'on souhaite mettre en place une stimulation de type double chambre il est nécessaire de disposer d'électrodes au niveau de l'oreillette pour assurer les fonctions requises de détection et/ou de stimulation atriale.

L'implantation d'une sonde conventionnelle reliée à un boîtier de générateur distant et munie d'électrodes au niveau de l'oreillette et du ventricule ne pose pas de difficulté particulière. En revanche, avec la technique leadless il est nécessaire d'implanter une première capsule dans le ventricule et une seconde capsule dans l'oreillette, les deux capsules étant munies de moyens de communication mutuelle pour assurer les fonctions requises de la stimulation double chambre.

La réduction de la longueur de la capsule est donc un préalable indispensable à la réalisation d'une capsule leadless auriculaire implantable.

Mais si l'on alimente une telle capsule par un récupérateur de type PEH, la présence de la lame PZT, qui doit être assez longue pour permettre un débattement suffisant pour produire assez de charges électriques afin d'alimenter convenablement les circuits de la capsule, implique pour la capsule un facteur de forme en longueur, qui est celui typiquement rencontré sur la quasi-totalité des capsules décrites par l'état de la technique, avec une forme de tube allongé.

Ce problème de la recherche d'une compacité accrue n'est toutefois pas spécifique aux capsules leadless cardiaques, auriculaires ou autres, mais se pose chaque fois que l'on veut miniaturiser un système PEH, quelle que soit l'application envisagée.

De façon générale, dans une optique de miniaturisation poussée et d'intégration du design d'un PEH, il est souhaitable de réduire le volume globalement nécessaire à l'oscillation de la lame PZT, qui est un volume perdu, dans lequel il n'est pas possible de placer des composants électriques, électroniques ou mécaniques, ou qui permettrait de disposer d'une batterie un peu plus volumineuse procurant une capacité accrue. Dans cette même optique, il serait également souhaitable qu'un même élément de structure puisse assurer plusieurs fonctions, afin de réduire le volume global du système, en faciliter l'industrialisation et réduire le coût de fabrication.

### RÉSUMÉ DE L'INVENTION

Pour résoudre les différents problèmes et atteindre les buts exposés ci-dessus, l'invention propose un module de récupération d'énergie de type PEH comprenant, de manière en elle-même connue, notamment d'après le WO 2019/001829 A1 précité, un ensemble pendulaire soumis à des sollicitations externes appliquées au module, l'ensemble pendulaire comprenant un transducteur piézoélectrique élastiquement déformable en flexion avec une extrémité encastrée et une extrémité libre couplée à une masse inertielle, le transducteur piézoélectrique étant apte à convertir une énergie mécanique produite par des oscillations de l'ensemble pendulaire en un signal électrique oscillant recueilli par des électrodes de surface du transducteur piézoélectrique. l'invention est définie par les revendications indépendantes 1 et 11.

Selon un *aspect* de l'invention, le transducteur piézoélectrique comprend au moins une lame piézoélectrique configurée en deux branches attenantes formées d'un seule pièce, avec une branche externe et une branche interne agencées côte-à-côte, la branche externe ayant une extrémité proximale fixée à un encastrement et une extrémité distale libre, et la branche interne ayant une extrémité proximale libre et supportant la masse inertielle, et une extrémité distale libre et réunie à l'extrémité distale de la branche externe. Le module comprend en outre une monture annulaire qui entoure la lame piézoélectrique à son extrémité proximale et qui comprend ledit encastrement auquel est fixée l'extrémité proximale de la branche externe, et la monture annulaire comporte, dans une région centrale au voisinage de l'encastrement, une cavité à l'intérieur de laquelle peut osciller la masse inertielle portée par l'extrémité proximale libre de la branche interne.

Selon diverses formes de réalisation avantageuses :
- la monture annulaire comprend un limiteur de course comportant à l'intérieur de la cavité des surfaces de limitation de course aptes à former une butée pour la masse inertielle dans une configuration de flexion maximale de la lame piézoélectrique ;
- la masse inertielle comprend des surfaces de limitation de course complémentaires des surfaces de limitation de course en vis-à-vis de la monture annulaire ;
- le contact mutuel entre les surfaces de limitation de course de la monture annulaire et les surfaces de limitation de course de la masse inertielle est l'un d'entre : un contact plan, un contact linéaire, ou un contact surfacique-cylindrique ;
- les surfaces de limitation de course de la monture annulaire et/ou de la masse inertielle sont revêtues d'un matériau souple amortisseur, ou réalisées en un matériau amortisseur ;
- le transducteur piézoélectrique comprend deux lames piézoélectriques coplanaires agencées côte-à-côte, chacune des lames piézoélectriques comprenant une dite branche externe et une dite branche interne, les extrémités proximales des branches internes des deux lames piézoélectriques supportent ensemble la masse inertielle, et les extrémités proximales des branches externes des deux lames piézoélectriques sont liées chacune à un encastrement respectif de la monture annulaire, les deux encastrements étant situés de part et d'autre de la masse inertielle dans la cavité ;
- la position du centre de gravité de la masse inertielle est située à l'intérieur de la cavité de la monture annulaire ;
- suivant une direction axiale de la lame dans sa plus grande dimension, la position du centre de gravité de la masse inertielle est décalée dans un sens proximal par rapport au centre de la cavité de la monture annulaire ;
- la lame piézoélectrique porte à son extrémité distale libre une masselotte intermédiaire à l'endroit de la jonction entre la branche externe et la branche interne ; et/ou
- le module comprend en outre, au voisinage de l'extrémité distale de la lame piézoélectrique, un limiteur de course apte à former une butée pour la masselotte intermédiaire dans une configuration de flexion maximale de la lame piézoélectrique.

L'invention a également pour objet un dispositif autonome logeant, dans un corps de dispositif : un ensemble électronique ; un module de récupération d'énergie tel que ci-dessus, produisant en sortie un signal électrique oscillant ; un circuit de gestion d'alimentation, apte à redresser et réguler le signal électrique oscillant produit par le module de récupération d'énergie, pour délivrer en sortie une tension ou un courant continus d'alimentation stabilisés ; et un organe de stockage d'énergie pour l'alimentation de l'ensemble électronique. Ladite tension ou ledit courant continus stabilisés sont délivrés par le circuit de gestion d'alimentation servent à l'alimentation de l'ensemble électronique et/ou à la recharge de l'organe de stockage d'énergie du dispositif autonome.

Le dispositif autonome peut en particulier être un dispositif médical actif de type capsule autonome implantable comprenant un corps de capsule avec un élément d'ancrage à une paroi d'un organe d'un patient, les sollicitations externes auxquelles est soumis l'ensemble pendulaire du module de récupération d'énergie étant des sollicitations appliquées au corps de capsule sous l'effet de mouvements de ladite paroi et/ou de variations de débit d'un flux dans le milieu environnant.

### DESCRIPTION SOMMAIRE DES DESSINS

On va maintenant décrire un exemple de réalisation de la présente invention en référence aux dessins annexés, où les mêmes références désignent d'une figure à l'autre des éléments identiques ou fonctionnellement semblables.
La Figure 1 illustre des dispositifs médicaux de type capsule leadless dans leur environnement, avec divers exemples de sites d'implantation dans, sur ou à proximité du coeur d'un patient.
La Figure 2 illustre une capsule leadless implantée au fond du ventricule droit d'un patient.
La Figure 3 montre, isolément un ensemble pendulaire de type connu, avec un PZT en forme de lame allongée encastrée à une extrémité et supportant une masse inertielle à son extrémité opposée.
La Figure 4 présente sous forme schématique les principaux blocs fonctionnels constitutifs d'une capsule leadless.
La Figure 5 est une vue en coupe montrant, avec une échelle des épaisseurs volontairement exagérée, la structure des différentes couches d'un PZT bimorphe utilisable avec la présente invention.
La Figure 6 est une vue en plan d'un PZT selon l'invention, sous forme de deux lames agencées côte à côte avec pour chacune deux branches parallèles interne et externe.
La Figure 7 est homologue de la Figure 6, pour une variante avec une courbure non arrondie à la jonction entre les deux branches interne et externe.
La Figure 8 est une vue perspective du PZT de la Figure 6, muni d'électrodes de surface sur chacune des faces inférieure et supérieure de chaque branche externe.
La Figure 9 est homologue de la Figure 8, pour une variante où les branches externes et également internes sont munies d'électrodes de surface.
La Figure 10 est un schéma électrique simplifié montrant la manière de coupler les différentes électrodes de surface du PZT de la Figure 9 à des redresseurs afin de débiter en sortie un courant de recharge d'une batterie tampon.
La Figure 11 présente une série de chronogrammes montrant les variations instantanées de l'accélération du PZT et les divers signaux électriques recueillis par les électrodes avant et après redressement et couplage mutuel.
La Figure 12 illustre une variante du PZT de l'invention, dans laquelle les deux branches internes sont réunies en une unique branche commune aux deux lames.
La Figure 13 est une vue en perspective montrant le PZT de la Figure 9 équipé d'une masse inertielle d'extrémité et fixé à une monture qui définit les encastrements des lames et assure en outre une fonction de limitation de course.
La Figure 14 est une vue en coupe schématique de l'ensemble de la Figure 13, avec le PZT mobile en position centrale.
La Figure 15 est homologue de la Figure 14, en configuration de déformation maximale du PZT avec butée de la masse inertielle contre une surface interne de la monture.
La Figure 16 est homologue de la Figure 15, pour une variante dans laquelle la masse inertielle est de forme externe plane et la surface homologue interne de butée de la monture est inclinée.
La Figure 17 est une vue en coupe transversale de la monture de la Figure 16, montrant le contact plan en butée de la masse inertielle contre la surface interne homologue de la monture.
La Figure 18 est homologue de la Figure 17, pour une variante dans laquelle le contact en butée est un contact linéaire axial.
La Figure 19 est homologue de la Figure 17, pour une autre variante dans laquelle le contact en butée est un contact surfacique-cylindrique.
La Figure 20 est homologue de la Figure 15, pour un contact de la masse inertielle contre une face interne de la monture revêtue d'une couche de polymère ou de silicone.
La Figure 21 est homologue de la Figure 16, pour un contact de masse inertielle contre une face interne de la monture revêtue d'une couche de polymère ou de silicone.
La Figure 22 est homologue de la Figure 14, pour une variante dans laquelle le montage de la masse inertielle à la lame est un montage soudé, sans collage.
La Figure 23 est homologue de la Figure 13, pour un mode de réalisation dans lequel une masselotte intermédiaire est ajoutée sur une face du PZT, au niveau de la jonction entre les branches internes et externes.
La Figure 24 est homologue de la Figure 23, pour une variante où des masselottes sont ajoutées sur chacune des faces supérieure et inférieure du PZT.
La Figure 25 est une vue en coupe homologue de la Figure 15, pour la variante de la Figure 24.
La Figure 26 est homologue de la Figure 23, pour une variante dans laquelle des masselottes distinctes sont ajoutées sur une face du PZT, séparément pour chacune des lames du PZT.
La Figure 27 est homologue de la Figure 26, pour une variante dans laquelle les masselottes sont ajoutées sur chacune des faces supérieure et inférieure du PZT.
La Figure 28 est homologue de la Figure 27, pour une variante dans laquelle les deux lames du PZT ont des longueurs respectives différentes.

### DESCRIPTION DÉTAILLÉE DE MODES DE RÉALISATION PRÉFÉRENTIELS DE L'INVENTION

On va maintenant décrire un exemple de réalisation du dispositif de l'invention, dans une application à une capsule implantable autonome destinée à être implantée dans une cavité cardiaque.

Comme on l'a indiqué plus haut, cette application particulière n'est donnée qu'à titre d'exemple de réalisation et n'est pas limitative de l'invention, dont les enseignements peuvent être appliqués à de nombreux autre types de dispositifs autonomes incorporant un récupérateur d'énergie de type PEH, que ces dispositifs soient implantables ou non, qu'il s'agisse de dispositifs médicaux ou non.

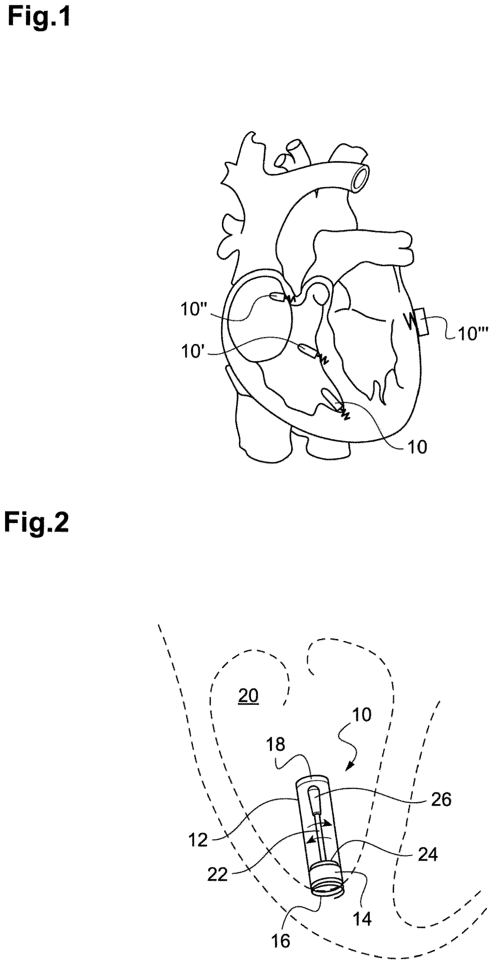

Sur la Figure 1, on a représenté diverses possibilités de sites d'implantation d'un dispositif de type leadless, dans une application à la stimulation cardiaque. Ainsi, la capsule 10 est implantée à l'intérieur d'une cavité du myocarde (implant endocavitaire), par exemple à l'apex du ventricule droit. En variante, la capsule peut être également implantée sur le septum interventriculaire droit, comme en 10', ou encore sur une paroi auriculaire, comme illustré en 10". Le dispositif peut également être une capsule épicardique placée sur une région externe du myocarde, comme illustré en 10‴.

Dans chaque cas, la capsule leadless est fixée à la paroi cardiaque au moyen d'un système d'ancrage saillant pénétrant dans le tissu cardiaque pour le maintien sur le site d'implantation. D'autres systèmes d'ancrage sont utilisables, et ne modifient en aucune façon la mise en oeuvre de la présente invention.

La capsule 10 se présente sous forme extérieure d'un implant avec un corps tubulaire allongé 12 enfermant les divers circuits électroniques et d'alimentation de la capsule ainsi qu'un récupérateur d'énergie à ensemble pendulaire. Les dimensions typiques des capsules connues sont un diamètre de l'ordre de 6 mm pour une longueur d'environ 25 à 40 mm.

Le corps tubulaire 12 comporte à son extrémité avant (distale) 14 un élément d'ancrage saillant, par exemple une vis hélicoïdale 16 pour maintenir la capsule sur le site d'implantation. D'autres systèmes d'ancrage sont utilisables, et ne modifient en aucune façon la mise en oeuvre de la présente invention. L'extrémité opposée (proximale) 18 de la capsule 10 est une extrémité libre, qui est seulement pourvue de moyens (non représentés) de liaison temporaire à un cathéter-guide, ou autre accessoire d'implantation utilisé pour la mise en place ou l'explantation de la capsule, qui est ensuite désolidarisé de cette dernière.

Dans l'exemple illustré Figure 2, la capsule leadless 10 est un implant endocavitaire implanté à l'intérieur d'une cavité 20 du myocarde, par exemple à l'apex du ventricule droit. En variante, toujours dans une application à la stimulation cardiaque, la capsule peut être également implantée sur le septum interventriculaire ou sur une paroi auriculaire, ou encore être une capsule épicardique placée sur une région externe du myocarde, ces différents modes d'implantation ne modifiant en aucune façon la mise en oeuvre de la présente invention. Pour assurer les fonctions de détection/stimulation, une électrode (non représentée) en contact avec le tissu cardiaque au site d'implantation assure le recueil des potentiels de dépolarisation cardiaque et/ou l'application d'impulsions de stimulation. Dans certaines formes de réalisation, la fonction de cette électrode peut être assurée par la vis d'ancrage 16, qui est alors une vis active, électriquement conductrice et reliée au circuit de détection/stimulation de la capsule.

La capsule leadless 10 est par ailleurs dotée d'un module récupérateur d'énergie dit "PEH", comprenant un ensemble pendulaire inertiel qui oscille à l'intérieur de la capsule au gré des diverses sollicitations externes auxquelles la capsule est soumise. Ces sollicitations peuvent notamment résulter : des mouvements de la paroi à laquelle est ancrée la capsule, qui sont transmis au corps tubulaire 12 par la vis d'ancrage 16 ; et/ou des variations du débit du flux sanguin dans le milieu environnant la capsule, qui produisent des oscillations du corps tubulaire 12 au rythme des battements cardiaques ; et/ou des vibrations diverses transmises par les tissus cardiaques.

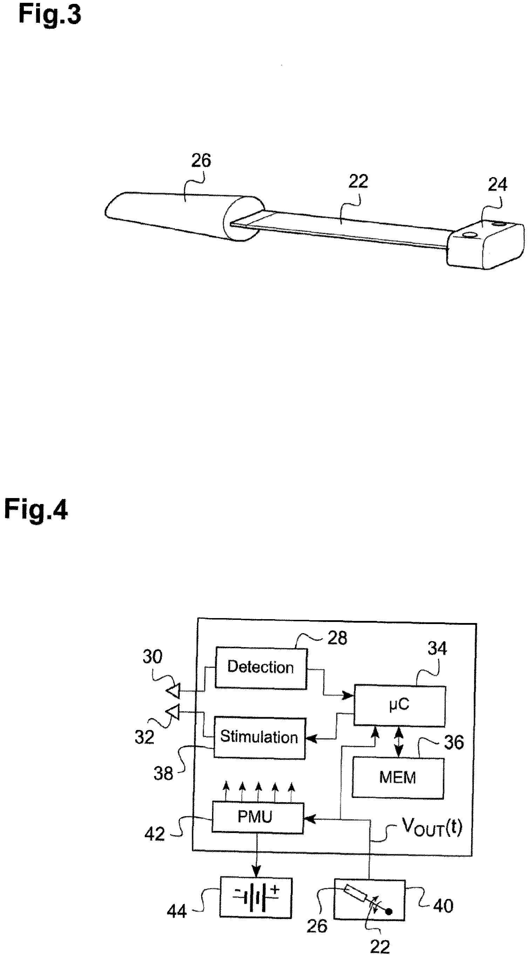

L'ensemble pendulaire, illustré isolément Figure 3, est constitué d'une lame piézoélectrique 22 encastrée en 24 à l'une de ses extrémités et dont l'extrémité opposée, libre, est couplée à une masse inertielle mobile 26. La lame piézoélectrique 22 est une lame flexible élastiquement déformable qui constitue avec la masse inertielle 26 un système pendulaire de type masse-ressort. Du fait de son inertie, la masse 26 soumet la lame 22 à une déformation de type vibratoire de part et d'autre d'une position neutre ou non déformée correspondant à une position stable de repos en l'absence de toute sollicitation. Les dimensions typiques minimales des lames PZT des dispositifs connus de ce type sont de l'ordre de 25 mm de long pour une largeur de 5 mm environ.

De fait, pour ce qui est de son comportement mécanique, cet ensemble peut être assimilé à une structure de type "poutre encastrée-libre", présentant une fréquence d'oscillation propre qui est ici la fréquence sur laquelle oscille le système masse-ressort. On notera que cette fréquence d'oscillation propre, typiquement de l'ordre de quelques dizaines de hertz, est notablement supérieure à la fréquence des sollicitations cycliques externes qui correspondent à la fréquence des battements cardiaques (quelques hertz tout au plus). Ainsi, à chaque contraction cardiaque la masse inertielle (ou autre organe mécanique fonctionnellement analogue) sera sollicitée avec une amplitude plus ou moins importante, puis le système pendulaire oscillera plusieurs fois avec des amplitudes décroissantes (rebonds caractéristiques d'une oscillation périodique amortie), et enfin se stabilisera jusqu'au battement cardiaque suivant, où le cycle sollicitation/oscillations se reproduira de façon comparable.

La lame 22 assure en outre, par effet piézoélectrique, une fonction de transducteur mécanoélectrique permettant de convertir en charges électriques la contrainte mécanique de flexion qui lui est appliquée. Ces charges sont collectées par des électrodes à la surface de la lame de manière à produire un signal électrique qui, après redressement, stabilisation et filtrage, alimentera les divers circuits électroniques de la capsule.

La Figure 4 est un synoptique des divers circuits électriques et électroniques intégrés à la capsule leadless, présenté sous forme de blocs fonctionnels.

Le bloc 28 désigne un circuit de détection de l'onde de dépolarisation cardiaque, qui est relié à une électrode de cathode 30 en contact avec le tissu cardiaque et à une électrode d'anode 32 associée, par exemple une électrode annulaire formée sur le corps tubulaire de la capsule. Le bloc de détection 28 comprend des filtres et des moyens de traitement analogique et/ou numérique du signal recueilli. Le signal ainsi traité est appliqué à l'entrée d'un microcalculateur 34 associé à une mémoire 36. L'ensemble électronique comporte également un circuit de stimulation 38 opérant sous le contrôle du microcalculateur 34 pour délivrer au système d'électrodes 30, 32 des impulsions de stimulation du myocarde.

Il est par ailleurs prévu un circuit récupérateur d'énergie ou PEH 40, constitué par l'ensemble pendulaire formé par la lame piézoélectrique 22 et la masse inertielle 26 décrits plus haut en référence aux Figures 2 et 3. Comme la lame piézoélectrique 22 assure aussi une fonction de transducteur mécano-électrique, elle convertit en charges électriques les sollicitations mécaniques subies et produit un signal électrique variable V_{OUT}(t), qui est un signal alternatif oscillant à la fréquence d'oscillation libre de l'ensemble pendulaire lame 22/masse 26, et au rythme des battements successifs du myocarde auquel la capsule est couplée.

Le signal électrique variable V_{OUT}(t) est délivré à un circuit de gestion de l'énergie ou PMU 42. Le PMU 42 redresse et régule le signal V_{OUT}(t) de manière à produire en sortie une tension ou un courant continus stabilisés servant à l'alimentation des divers circuits électroniques et à la recharge de la batterie intégrée 44.

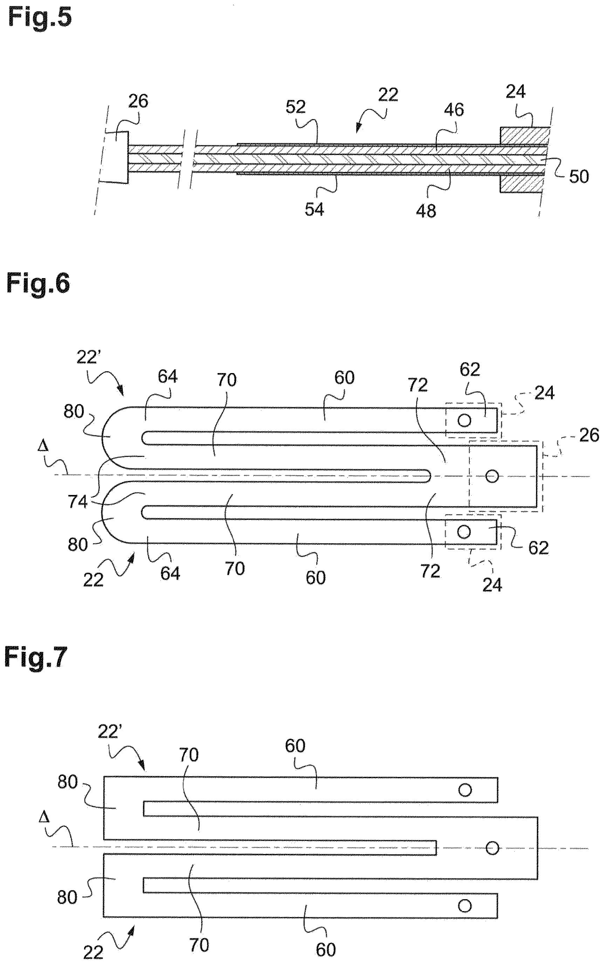

D'autre part, la lame est avantageusement une lame de type bimorphe, c'est-à-dire capable de générer de l'énergie sur ses deux faces lorsqu'elle est soumise à une déformation. Ces propriétés de transduction sont typiques d'un matériau piézoélectrique tel que les céramiques PZT ou les monocristaux de type PMN-PT, titanate de baryum ou niobate de lithium. La Figure 5 illustre de façon schématique, avec une échelle des épaisseurs volontairement exagérée, la structure des différentes couches d'une telle lame PZT bimorphe.

La lame 22 bimorphe comprend deux couches 46, 48 de matériau céramique PZT déposées sur chacune des faces opposées d'une âme centrale ou *shim* 50 en matériau conducteur (ou en variante en un matériau isolant, avec des ponts de contact permettant de shunter les électrodes internes des couches piézoélectriques). Cette structure bimorphe correspond en fait à l'association de deux structures unimorphes placées dos-à-dos, avec mise en commun de l'âme supportant le matériau PZT. Si l'âme 50 est en matériau conducteur, il est possible de recueillir les charges produites par la déformation du matériau PZT aussi bien entre l'âme conductrice 50 et une électrode de surface 52, 54 de l'un et/ou de l'autre des PZT 46, 48, qu'entre les deux électrodes de surface 52, 54 des faces opposées de la lame, indépendamment de l'âme centrale.

La Figure 6 est une vue en plan d'un PZT selon l'invention, considéré isolément avant réalisation des encastrements et mise en place de la masse inertielle.

De façon caractéristique de l'invention, ce PZT est formé de deux lames piézoélectriques 22, 22' coplanaires agencées côte à côte, de part et d'autre d'un axe central Δ correspondant à la direction de plus grande longueur du PZT.

Chacune des deux lames 22, 22' présente une forme repliée avec deux branches 60, 70 attenantes, agencées côte à côte et formées d'une seule pièce. La branche externe 60 a son extrémité proximale 62 encastrée (encastrement 24) et son extrémité distale 64 libre, tandis que la branche interne 70 a son extrémité proximale 72 qui est libre en déplacement et qui supporte la masse inertielle 26, et son extrémité distale 74 qui est libre (ici et dans la suite, le côté "proximal" sera entendu comme étant celui qui est lè plus proche de l'encastrement de la lame, et le côté "distal" le côté opposé, le plus éloigné de l'encastrement). L'extrémité distale 64 de la branche externe 60 et l'extrémité distale 74 de la branche interne 70 sont réunies ensemble par une jonction commune 80.

Le PZT constitué des deux lames 22, 22' avec pour chacune les branches internes et externes que l'on vient de décrire peut être notamment réalisé par découpe au laser dans une unique pièce de matériau.

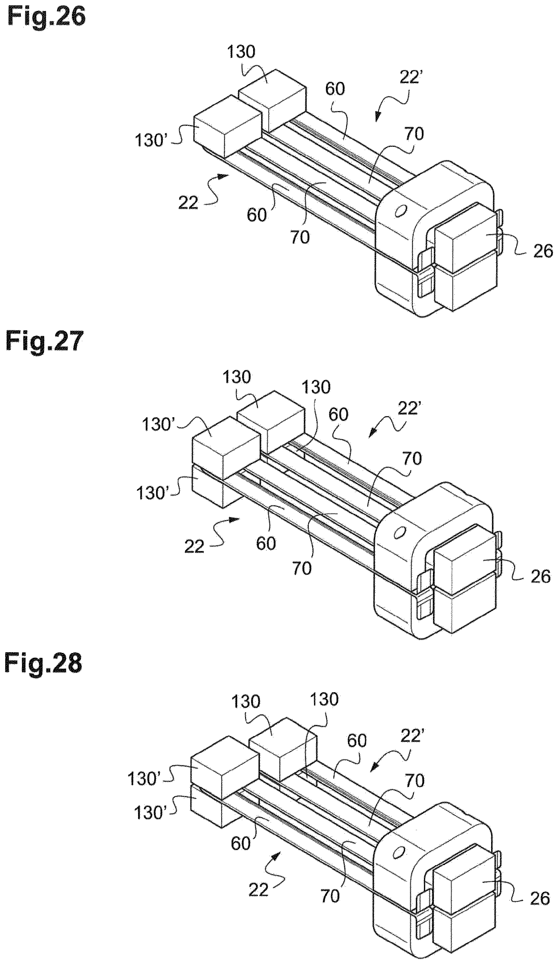

Les deux lames 22, 22' peuvent être symétriques (comme illustré) ou non, de même longueur (comme illustré) ou non (la Figure 28 montre en particulier un exemple où les deux lames 22, 22' ont des longueurs différentes).

Pour chaque lame, les branches externe et interne 60, 70 peuvent être approximativement parallèles (comme illustré) ou non, avoir des bords parallèles (comme illustré) ou non, et être de même longueur et/ou largeur (comme illustré) ou non.

Comme cela est décrit dans la demande EP 20315317.6 du 24.06.2020 pour *"Module de récupération d'énergie à transducteur piézoélectrique, notamment pour la recharge optimisée de la batterie d'un dispositif médical implantable tel qu'une capsule cardiaque autonome leadless",* afin de renforcer la fiabilité des lames PZT 22, 22', il est possible de donner à celles-ci, sur tout ou partie de leur longueur, une forme en plan trapézoïdale avec une décroissance (linéaire ou exponentielle) de la largeur pour mieux répartir les contraintes le long de la lame, ces contraintes étant plus fortes à proximité et au niveau de l'encastrement 24, et nulles au niveau de la masse inertielle 26. De plus, la forme trapézoïdale permet d'ajuster la fréquence de résonance de l'ensemble en fonction de la géométrie du trapèze, tout en maximisant l'amplitude de déplacement de la masse du fait que l'extrémité libre est plus étroite que l'extrémité encastrée.

Avec la configuration que l'on vient de décrire, chacune des deux lames 22, 22' présente un double porte-à-faux correspondant à chacune des deux branches externe et interne 60, 70, à savoir : un premier porte-à-faux pour la branche externe 60 entre l'encastrement 24 à l'extrémité proximale 62 et l'extrémité distale 64 qui est libre d'osciller ; et un second porte-à-faux pour la branche interne 70 entre l'extrémité distale 74 liée à l'extrémité distale 64 (mobile) de la branche externe 60 et l'extrémité proximale 72 portant la masse inertielle 26.

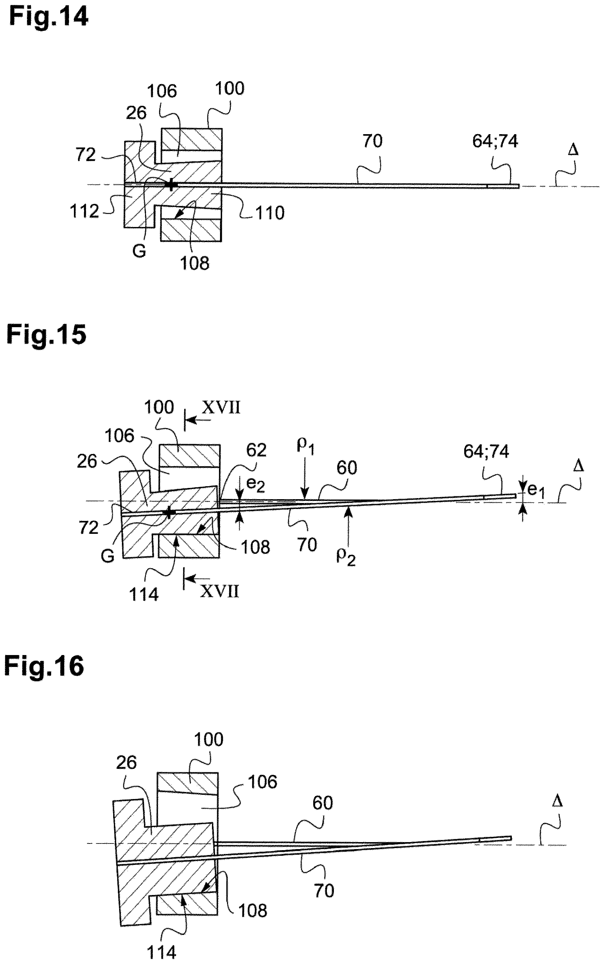

Les oscillations de l'ensemble pendulaire constitué par l'encastrement 24, la lame 22 et la masse inertielle 26 produisent des déplacements respectifs des deux branches 60, 70 dans des sens respectifs opposés (on pourra se référer plus loin à la Figure 15 qui montre, en élévation et en coupe, les flexions respectives opposées de ces deux branches).

Du point de vue de l'encombrement global du transducteur, cette configuration particulière en deux branches parallèles permet de diminuer dans des proportions importantes la longueur hors-tout du PZT (dimension considérée dans la direction longitudinale de l'axe Δ) par rapport à une lame conventionnelle telle que celle illustrée Figure 3, le gain de longueur étant typiquement d'au moins 20 à 40 %.

D'autre part, avec le double porte-à-faux et les déplacements des deux branches dans des sens contraires, l'amplitude hors-tout de l'oscillation de la lame sera réduite par rapport à un PZT conventionnel du fait que les courses respectives de chacune des deux branches 60, 70 s'additionnent en valeur absolue mais sont en sens contraire. Ceci permet de réduire également dans des proportions importantes le volume nécessaire au débattement de l'ensemble pendulaire par rapport à une configuration conventionnelle, avec une diminution corrélative du volume mort qui, en pratique, est une place perdue qui n'est pas utilisable pour y placer des composants de circuit ou loger une batterie plus volumineuse.

La Figure 7 illustre une variante de la Figure 6 dans laquelle la jonction 80 entre les branches externe et interne 60, 70 n'est pas arrondie, mais rectangulaire. La configuration arrondie de la Figure 6 permet toutefois de mieux répartir les contraintes au niveau de la jonction 80 entre les deux branches, contraintes résultant du fait que ces deux branches fléchissent dans des sens opposés alors même qu'elles sont formées d'une seule pièce.

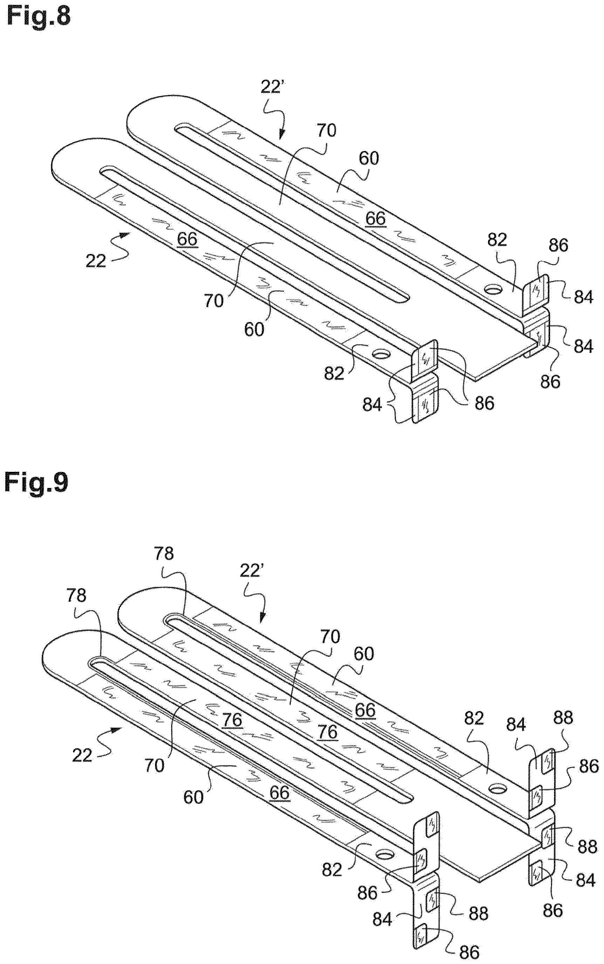

Le recueil des charges électriques produites par les flexions des branches lors des oscillations est assuré par un système d'électrodes illustré sur les vues en perspectives des Figures 8 et 9.

Sur la Figure 8 on a représenté un mode de réalisation dans lequel les branches externes 60 de chaque lame 22, 22' sont munies d'électrodes de surface 66 s'étendant sur la majeure partie de la longueur de la branche. Ces électrodes sont par exemple réalisées par un dépôt d'or, de cuivre, de platine ou de tout autre matière conductrice, et sont reliées électriquement à l'extrémité proximale de la branche à un circuit souple ou *flex* 82 se prolongeant par une partie libre relevée 84 portant une plage de connexion ou *pad* 86 électriquement reliée à l'électrode 66 et permettant le transfert des charges électriques produites par cette dernière vers un circuit électrique d'alimentation.

Si la lame utilisée est une lame bimorphe telle que celle décrite plus haut à propos de la Figure 5, la face inférieure opposée (non visible sur la figure) de la branche externe 60 est munie d'une électrode semblable, reliée à un autre pad 86 à l'extrémité proximale de la même branche. Les électrodes en vis-à-vis sur des faces opposées recueillent alors des charges de signes contraires, conduites aux pads 86 correspondants. L'âme centrale de la lame bimorphe (référencée 50 sur la Figure 5) peut comporter également une liaison électrique à un pad correspondant, utilisée comme potentiel de référence électrique de "neutre".

Avec une lame bimorphe, la configuration de la Figure 8 comporte ainsi au total quatre électrodes 66 (deux électrodes de chaque côté de chaque branche externe 60), quatre flex 82 et quatre pads 86.

Par ailleurs, comme cela est décrit dans la demande EP 20315317.6 précitée, il est possible de fractionner les électrodes de surface, ou certaines d'entre elles, sur la face supérieure et/ou sur la face inférieure de la lame, en deux ou plus sous-électrodes électriquement isolées entre elles. Ces sous-électrodes fractionnaires, qui peuvent être ou non symétriques par rapport à un axe longitudinal central de la lame, sont alors reliées chacune à un pad respectif distinct pour la prise de contact et la liaison à des étages redresseurs en aval.

La Figure 9 illustre un autre mode de réalisation dans lequel, outre les électrodes 66 sur les branches externes 60 de la Figure 8, le PZT comporte également des électrodes 76 sur les branches internes 70. Dans la mesure où les branches internes 76 se déplacent dans un sens opposé de celui des branches externes 60, les électrodes de surface respectives des branches interne et externe recueilleront des charges de signes opposés au cours d'une même déformation du PZT (charge positive sur les électrodes 66 et charge négative sur les électrodes 76, et *vice versa* à la demi-oscillation suivante). Comme il n'est pas possible pour cette raison de mettre en série les électrodes 76 et 66, chaque électrode 76 est reliée au flex 82 par un conducteur 78 qui court le long de la branche externe 60 en étant électriquement isolé de l'électrode 66, afin d'assurer une prise de connexion par un pad 88 sur la partie libre relevée d'extrémité 84 du flex, au voisinage des pads 86 reliés aux électrodes 66 des branches externes 60.

Avec une configuration d'électrodes semblable sur la face inférieure (non visible sur la figure), on obtient ainsi un agencement comprenant huit électrodes 66, 76, quatre flex 82 et huit pads 86, 88.

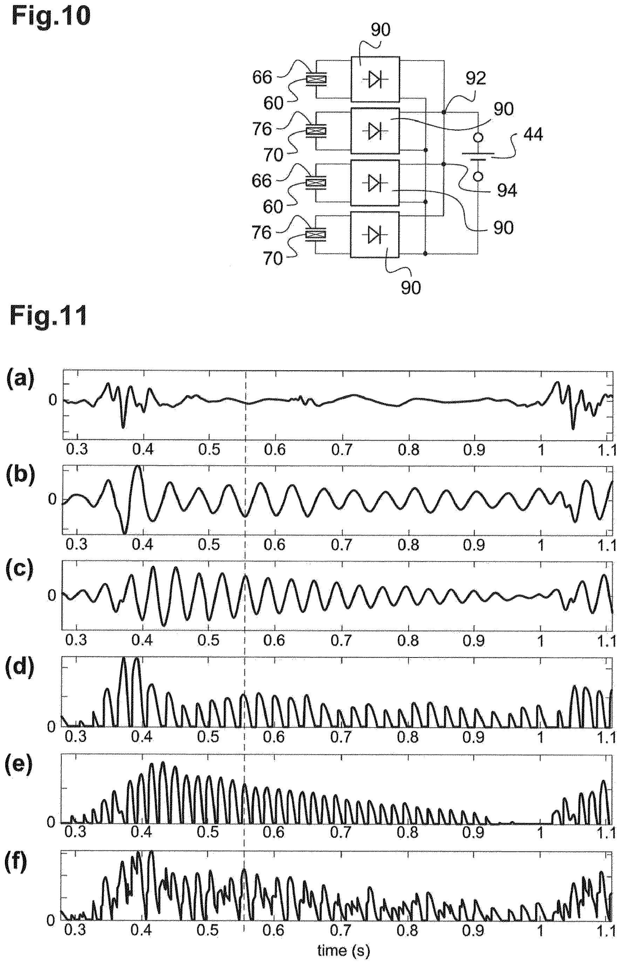

La Figure 10 est un schéma électrique simplifié montrant la manière de coupler entre elles les diverses électrodes de la configuration de la Figure 9 pour délivrer en sortie du PEH un courant d'alimentation et/ou de recharge d'une batterie tampon.

Chaque couple d'électrodes en face inférieure/supérieure de chacune des branches 60 ou 70 est relié à un circuit redresseur respectif 90, par exemple un redresseur double alternance FBR *(Full-Bridge Rectifier),* par exemple un pont de diodes (pont de Graetz), ou un convertisseur de tension négative NVC *(Négative Voltage Converter)* à MOSFET ou autre circuit analogue. Les sorties respectives des circuits redresseurs 90 sont reliées à une borne positive 92 commune et à une borne négative 94 commune délivrant entre elles un courant de recharge appliqué à la batterie tampon 44. Les circuits redresseurs 90 peuvent être éventuellement réalisés avec de composants disposés directement sur les flex 84 et reliés aux pads 86, 88 respectifs.

Avantageusement, les sorties correspondantes (positive ou bien négative) des redresseurs 90 sont directement reliées ensemble, sans couplage intermédiaire.

En variante, il est également possible de relier directement ensemble les diverses électrodes de surface recueillant des charges de même polarité, ces électrodes étant ensuite couplées à une même entrée respective d'un étage redresseur commun, tous les courants générés par le déplacement des charges de même signe s'additionnant pour être délivrés .à la batterie après redressement.

La Figure 11 est une série de chronogrammes montrant :
(a) les variations instantanées de l'accélération du PZT ;
(b) le courant généré par une paire d'électrodes supérieure/inférieure de l'une des branches externes 60 ;
(c) le courant généré par une paire d'électrodes supérieure/inférieure de l'une des branches internes 70 ;
(d) le courant (b), après redressement double alternance par un circuit 90 ;
(e) le courant (c), après redressement double alternance par un circuit 90 et
(f) le courant total résultant de l'addition des courants (b) et (e), injecté dans la batterie tampon 44 pour la recharge de cette dernière.

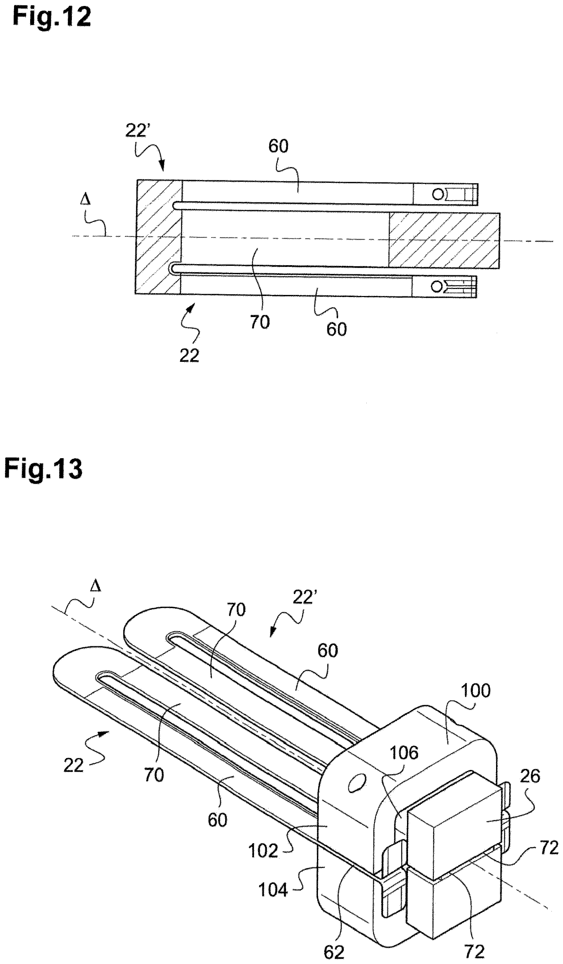

La Figure 12 illustre une variante de réalisation du PZT précédemment décrit en relation aux Figures 6 et 7, dans laquelle les deux branches internes 70 sont confondues en une branche unique sur la totalité de leur longueur (en variante, elles peuvent n'être confondues que sur une partie de leur longueur). Cette variante procure notamment au PZT une rigidité supérieure à l'endroit où se concentrent les contraintes lors des oscillations de l'ensemble pendulaire. En ce qui concerne la configuration des électrodes, la branche interne centrale 70 porte une électrode unique sur chacune des faces supérieure et inférieure, et chacune des deux branches externes 60 porte une électrode sur chacune de ses faces supérieure et inférieure. On dispose dans ce cas d'un ensemble comportant en tout six électrodes, avec six pads et quatre flex.

La Figure 13 illustre le PZT selon l'invention, tel qu'illustré notamment aux Figures 6 et 9, dans une configuration complète de l'ensemble pendulaire, avec les encastrements et la masse inertielle montée.

L'encastrement, au niveau des extrémités proximales 62 de chaque branche externe 60, est réalisé par une monture ou *clamp* 100, constituée ici de deux pièces distinctes 102, 104 assemblées de manière à prendre en sandwich les extrémités proximales 62 des branches externes 60, pour réaliser à cet endroit les encastrements 24 des deux lames 22, 22' du PZT.

La masse inertielle 26 est montée à l'extrémité proximale 72 des branches internes 70, et elle a la possibilité de se déplacer librement dans un espace libre 106 ménagé à cet endroit dans la monture 100, qui présente une forme sensiblement annulaire.

On soulignera que, outre la compacité accrue, cet agencement particulier selon l'invention permet de déporter le centre de gravité G de la masse inertielle 26 en arrière du centre de la monture 100, accroissant d'autant l'effet d'inertie.

Les Figures 14 et 15 montrent en coupe l'ensemble de la Figure 13, respectivement avec le PZT immobile en position centrale (donc en alignement sur l'axe Δ) et en configuration de déformation maximale. Dans ce dernier cas, les branches externes 60 sont déformées dans un premier sens (vers le haut sur la figure) avec une courbure *ρ₁* et une excursion e, par rapport à l'axe Δ, et les branches internes 70 se déforment dans le sens contraire avec une courbure ρ₂ opposée et une excursion maximale e₂ par rapport à l'axe Δ. On notera que la déformation totale du PZT correspond à la somme des deux excursions e₁ et e₂, avec l'avantage d'une déformation importante (|*e₁*|+|*e₂*|) dans un relativement faible volume de déplacement, par rapport à une configuration de PZT conventionnelle telle que celle illustrée Figure 3.

Avantageusement, la monture 100 comporte dans le volume interne 106 une surface interne 108 contre laquelle peut venir buter une surface externe en vis-à-vis 114 de la masse inertielle 26, ceci afin d'éviter une déformation excessive du PZT lors de fortes sollicitations et préserver ainsi la longévité de l'ensemble. Cette butée, par contact entre la masse inertielle et la monture, évite tout contact direct avec la lame, comme cela est le cas dans certains dispositifs de limitation de course connus, par exemple celui décrit par le US 2019/381325 A1 (Regnier et al.).

La Figure 16 illustre une variante de la Figure 15 dans laquelle la surface interne de butée 108 de la monture 100 est une surface inclinée par rapport à l'axe Δ (au lieu d'être parallèle à l'axe Δ comme dans le cas de la Figure 15), et la surface complémentaire 114 de la masse inertielle est une surface plane (au lieu d'être une surface inclinée comme dans le cas de la Figure 15).

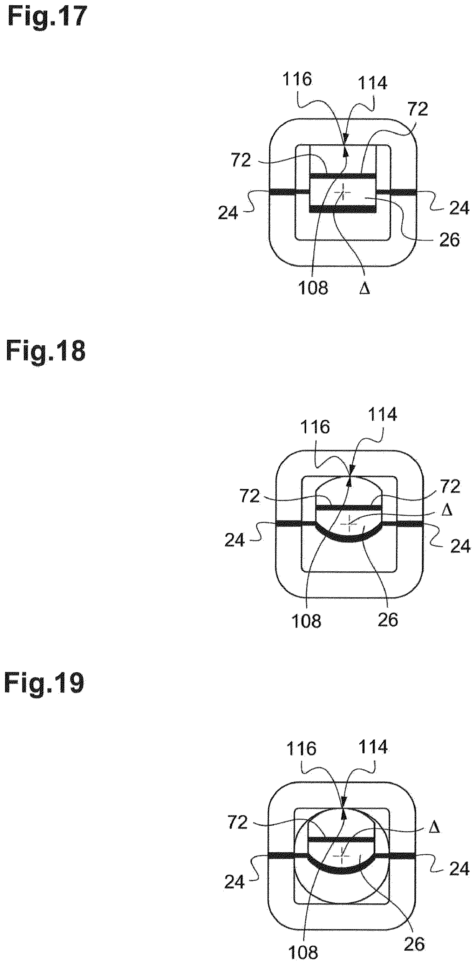

La Figure 17 est une vue en coupe transversale de la monture de la Figure 16, montrant le contact en section droite entre les surfaces complémentaires 108 et 114, qui est un contact plan 116 sur une étendue de surface relativement importante.

Dans une variante illustrée Figure 18, l'une des surfaces 108, 114 est une surface courbe et l'autre une surface plane, et le contact mutuel 118 est un contact linéaire, axial.

Dans un autre variante illustrée Figure 19, les surfaces 108 et 114 sont toutes deux des surfaces courbes, et le contact entre ces surfaces est un contact surfacique-cylindrique, qui procure une meilleure absorption des chocs du fait de la plus grande surface de contact mutuel.

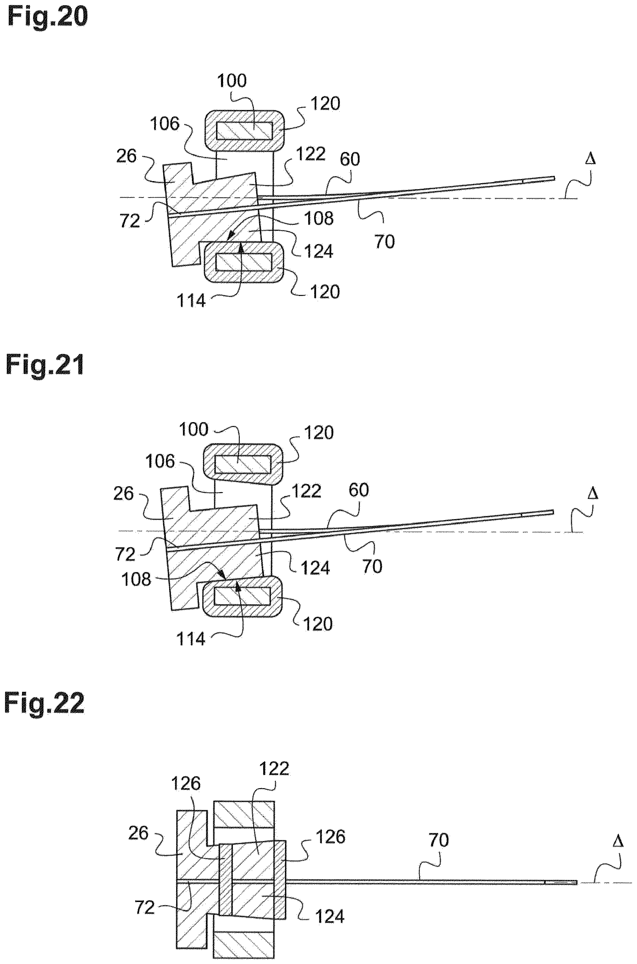

Les Figures 20 et 21 sont homologues des Figures 15 et 16, dans une variante où les zones de contact de la monture 100 sont couvertes d'un revêtement amortisseur approprié, par exemple un polymère ou un silicone, de manière à éviter un contact métal/métal entre la masse inertielle 26 et la monture 100 comme dans les exemples précédents.

La Figure 22 illustre une manière particulière de réaliser le montage de la masse inertielle 26 sur l'extrémité proximale 72 des branches 70.

Dans les exemples précédents, par exemple sur la Figure 20, la masse inertielle 26 est constituée de deux pièces distinctes 122, 124 collées sur l'extrémité 72 des branches internes 70, cette extrémité étant prise en sandwich entre les pièces 122 et 124. Le collage peut toutefois présenter un certain nombre de difficultés et de limitations si l'on veut garantir une fiabilité sans faille du dispositif pendant toute sa durée de vie (au moins 10 ans). Pour ces raisons, une construction non collée peut être préférable, par exemple une construction entièrement soudée telle que celle illustrée Figure 22, où les deux pièces 124, 126 sont réunies ensemble par des goupilles 126 soudées reliant entre elles les pièces 124, 126 en prenant en sandwich l'extrémité proximale 72 des branches internes 70.

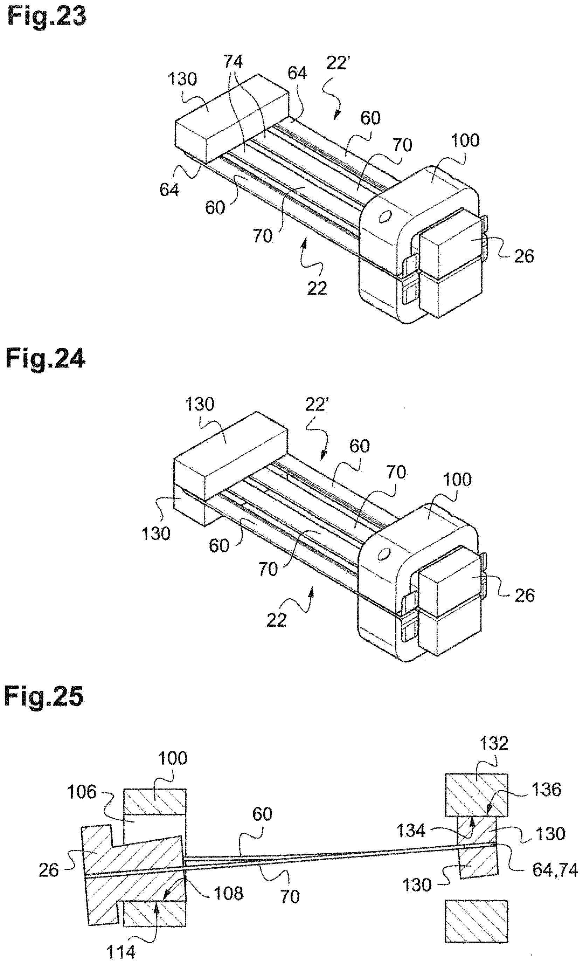

On va maintenant décrire, en référence aux Figures 23 à 28, divers modes de réalisation de l'invention mettant en oeuvre une masselotte intermédiaire 130 lestant les extrémités distales des branches externe et interne 60, 70 à l'endroit de leur jonction 80.

La présence d'une masselotte intermédiaire 130 à cet endroit présente plusieurs avantages :
- il s'agit d'un élément rigide qui supprime les contraintes à l'endroit des courbures 80 de jonction entre des branches internes 70 et externes 60, à un endroit où ces contraintes peuvent être particulièrement élevées ;
- la présence d'une masselotte 130 à cet endroit est sans incidence sur le rendement de conversion, dans la mesure où il s'agit d'une zone de la lame ne produisant pratiquement pas de charges (la flexion du matériau PZT étant minimale à cet endroit, qui ne porte d'ailleurs pas d'électrodes), donc sans incidence sur le rendement électrique du PEH ;
- la masselotte intermédiaire 130 constitue une masse mobile additionnelle pour les branches internes 70 qui accentue l'effet d'oscillation pendulaire pour ces dernières ;
- pour optimiser la production d'énergie par le transducteur, il est possible de dissocier les régimes de vibration des deux lames, comme on l'expliquera en référence aux Figures 26 à 28 ci-après.

Dans la variante de la Figure 23, une masselotte unique 130 est disposée, par exemple collée, sur la face supérieure des deux lames 22, 22' et est commune à ces deux lames.

Dans la variante de la Figure 24, une masselotte 130 est montée sur chacune des deux faces supérieure et inférieure des deux lames 22, 22', les masselottes 130 étant ici encore communes aux deux lames.

La Figure 25 est une vue en coupe de l'ensemble de la Figure 24, dans une configuration de déformation maximale du PZT. Avantageusement, une pièce 132 permet de limiter la déformation maximale du côté des extrémités distales 64, 74 des branches 60, 70, par venue en butée contre une surface interne 134 d'une surface externe homologue 136 de chacune des masselottes intermédiaires 130. Cet agencement assure une double limitation de la déformation du PZT, côté distal par les surfaces de butée 134 de la pièce 132, et côté proximal par les surfaces de butée 108 de la monture 100, à chaque fois sans contact direct avec le matériau de la lame.

Les variantes des Figures 26 et 27 sont homologues de celles des Figures 23 et 24, avec la masselotte intermédiaire 130 fractionnée en deux masselottes distinctes 130, 130', chacune pour l'une des deux lames 22, 22'. Les deux lames ne sont donc pas couplées aux extrémités distales des branches 60, 70, ce qui permet d'en dissocier le régime d'oscillation.

Les géométries et/ou les masses et/ou les masses volumiques des masselottes intermédiaires 130, 130' peuvent être choisies de manière à produire des régimes d'oscillation différents pour chacune des deux lames 22,. 22', pour optimiser la récupération d'énergie. De plus, outre les masselottes intermédiaires 130, 130', il est possible de donner aux deux lames des longueurs et/ou des largeurs différentes pour modifier leur mode de vibration, par exemple des longueurs différentes des branches comme cela est illustré avec les lames 22, 22' sur la Figure 28.

## Revendications

1. Un module de récupération d'énergie comprenant un ensemble pendulaire soumis à des sollicitations externes appliquées au module,
l'ensemble pendulaire comprenant un transducteur piézoélectrique (22) s'étendant, le long d'un axe central (Δ) correspondant à une direction de plus grande longueur du transducteur piézoélectrique, d'une extrémité distale à une extrémité proximale opposée, le transducteur étant élastiquement déformable en flexion entre une extrémité encastrée (24) et une extrémité libre couplée à une masse inertielle (26),
le transducteur piézoélectrique étant apte à convertir une énergie mécanique produite par des oscillations de l'ensemble pendulaire en un signal électrique oscillant (Vouï(t)) recueilli par des électrodes de surface du transducteur piézoélectrique,
dans lequel:
- le transducteur piézoélectrique comprend au moins une lame piézoélectrique (22) configurée en deux branches attenantes formées d'un seule pièce, avec une branche externe (60) et une branche interne (70) agencées côte-à-côte,
la branche externe (60) ayant une extrémité proximale (62) fixée à un encastrement (24) et une extrémité distale (64) libre, et la branche interne (70) ayant une extrémité proximale (72) libre et supportant la masse inertielle (26), et une extrémité distale (74) libre et réunie à l'extrémité distale (64) de la branche externe (60),
- le module comprend en outre autour de l'axe central (Δ) une monture annulaire (100) qui entoure la lame piézoélectrique à son extrémité proximale et qui comprend ledit encastrement (24) auquel est fixée l'extrémité proximale (62) de la branche externe (60), et
- la monture annulaire (100) comporte, dans une région centrale au voisinage de l'encastrement, une cavité (106) à l'intérieur de laquelle peut osciller transversalement à l'axe central (Δ) la masse inertielle (26) portée par l'extrémité proximale libre (72) de la branche interne (70).

2. Le module de la revendication 1, dans lequel la monture annulaire (100) comprend un limiteur de course comportant à l'intérieur de la cavité des surfaces de limitation de course (108) aptes à former une butée pour la masse inertielle (26) dans une configuration de flexion maximale de la lame piézoélectrique.

3. Le module de la revendication 2, dans lequel la masse inertielle (26) comprend des surfaces de limitation de course (114) complémentaires des surfaces de limitation de course (108) en vis-à-vis de la monture annulaire (100).

4. Le module de la revendication 3, dans lequel le contact mutuel entre les surfaces de limitation de course (108) de la monture annulaire (100) et les surfaces de limitation de course (114) de la masse inertielle (26) est l'un d'entre : un contact plan, un contact linéaire, ou un contact surfacique-cylindrique.

5. Le module de la revendication 2, dans lequel les surfaces de limitation de course (108 ; 114) de la monture annulaire (100) et/ou de la masse inertielle (26) sont revêtues d'un matériau souple amortisseur (120), ou réalisées en un matériau amortisseur.

6. Le module de la revendication 1, dans lequel :
- le transducteur piézoélectrique comprend deux lames piézoélectriques (22, 22') coplanaires agencées côte-à-côte, chacune des lames piézoélectriques comprenant une dite branche externe (60) et une dite branche interne (70),
- les extrémités proximales des branches internes (70) des deux lames piézoélectriques supportent ensemble la masse inertielle (26), et
- les extrémités proximales des branches externes (60) des deux lames piézoélectriques sont liées chacune à un encastrement respectif (26) de la monture annulaire (100), les deux encastrements étant situés de part et d'autre de la masse inertielle (26) dans la cavité.

7. Le module de la revendication 1, dans lequel la position du centre de gravité (G) de la masse inertielle (26) est située à l'intérieur de la cavité de la monture annulaire (100).

8. Le module de la revendication 1, dans lequel, suivant une direction axiale de la lame dans sa plus grande dimension, la position du centre de gravité (G) de la masse inertielle (26) est décalée dans un sens proximal par rapport au centre de la cavité de la monture annulaire (100).

9. Le module de la revendication 1, dans lequel la lame piézoélectrique porte à son extrémité distale libre une masselotte intermédiaire (130 ; 130') à l'endroit de la jonction (80) entre la branche externe (60) et la branche interne (70).

10. Le module de la revendication 9, dans lequel le module comprend en outre, au voisinage de l'extrémité distale de la lame piézoélectrique, un limiteur de course (132) apte à former une butée pour la masselotte intermédiaire (130 ; 130') dans une configuration de flexion maximale de la lame piézoélectrique.

11. Un dispositif autonome logeant, dans un corps de dispositif :
- un ensemble électronique (28-38) ;
- un module de récupération d'énergie (40) selon l'une des revendications précédentes, produisant en sortie un signal électrique oscillant ;
- un circuit de gestion d'alimentation (42), apte à redresser et réguler le signal électrique oscillant produit par le module de récupération d'énergie, pour délivrer en sortie une tension ou un courant continus d'alimentation stabilisés ; et
- un organe de stockage d'énergie (44) pour l'alimentation de l'ensemble électronique,
dans lequel ladite tension ou ledit courant continus stabilisés délivrés par le circuit de gestion d'alimentation servent à l'alimentation de l'ensemble électronique et/ou à la recharge de l'organe de stockage d'énergie du dispositif autonome.

12. Le dispositif autonome de la revendication 11,
dans lequel le dispositif autonome est un dispositif médical actif de type capsule autonome implantable (10) comprenant un corps de capsule (12) avec un élément d'ancrage (16) à une paroi d'un organe d'un patient,
et dans lequel les sollicitations externes auxquelles est soumis l'ensemble pendulaire (22, 26) du module de récupération d'énergie sont des sollicitations appliquées au corps de capsule (12) sous l'effet de mouvements de ladite paroi et/ou de variations de débit d'un flux dans le milieu environnant.

## Patentansprüche

1. Ein Energierückgewinnungsmodul, umfassend eine pendelartige Anordnung, die äußere Belastungen erfährt, die auf das Modul aufgebracht werden, wobei die pendelartige Anordnung einen piezoelektrischen Wandler (22) umfasst, der sich, entlang einer mittigen Achse (Δ), die einer Richtung der größten Länge des piezoelektrischen Wandlers entspricht, von einem distalen Ende zu einem gegenüberliegenden proximalen Ende erstreckt, wobei der Wandler zwischen einem eingespannten Ende (24) und einem freien Ende, das an eine inertiale Masse (26) gekoppelt ist, elastisch biegeverformbar ist, wobei der piezoelektrische Wandler imstande ist, eine mechanische Energie, die durch Schwingungen der pendelartigen Anordnung erzeugt werden, in ein elektrisches Schwingungssignal (V_{OUT}(t)) umzuwandeln, das von Oberflächenelektroden des piezoelektrischen Wandlers empfangen wird,
wobei:
- der piezoelektrische Wandler wenigstens ein piezoelektrisches Längsorgan (22) umfasst, dass als zwei Schenkel ausgestaltet ist, die sich anschließen und aus einem Stück gebildet sind, mit einem äußeren Schenkel (60) und einem inneren Schenkel (70), die nebeneinander angeordnet sind, wobei der äußere Schenkel (60) ein proximales Ende (62), das an einem Einspannelement (24) befestigt ist, und ein freies distales Ende (64) aufweist und wobei der innere Schenkel (70) ein freies proximales Ende (72), das die inertiale Masse (26) stützt, und ein freies distales Ende (74), das mit dem distalen Ende (64) des äußeren Schenkels (60), verbunden ist, aufweist,
- das Modul ferner um die mittige Achse (Δ) herum eine ringförmige Montagevorrichtung (100) umfasst, die das piezoelektrische Längsorgan an seinem proximalen Ende umgibt und die das Einspannelement (24), an dem das proximale Ende (62) des äußeren Schenkels (60) befestigt ist, umfasst, und
- die ringförmige Montagevorrichtung (100), in einer mittigen Region in der Nähe des Einspannelements, einen Hohlraum (106) aufweist, in dessen Innerem die inertiale Masse (26) quergerichtet zu der mittigen Achse (Δ) schwingen kann, die von dem freien proximalen Ende (72) des inneren Schenkels (70) getragen wird.

2. Das Modul nach Anspruch 1, wobei die ringförmige Montagevorrichtung (100) einen Hubbegrenzer umfasst, der im Inneren des Hohlraums Hubbegrenzungsflächen (108) aufweist, die imstande sind, einen Anschlag für die inertiale Masse (26) in einer maximalen Biegekonfiguration des piezoelektrischen Längsorgans zu bilden.

3. Das Modul nach Anspruch 2, wobei die inertiale Masse (26) zu den Hubbegrenzungsflächen (108) komplementäre Hubbegrenzungsflächen (114) gegenüber der ringförmigen Montagevorrichtung (100) umfasst.

4. Das Modul nach Anspruch 3, wobei der gegenseitige Kontakt zwischen den Hubbegrenzungsflächen (108) der ringförmigen Montagevorrichtung (100) und den Hubbegrenzungsflächen (114) der inertialen Masse (26) einer ist von: einem flächigen Kontakt, einem linearen Kontakt oder einem zylinderoberflächigen Kontakt.

5. Das Modul nach Anspruch 2, wobei die Hubbegrenzungsflächen (108; 114) der ringförmigen Montagevorrichtung (100) und/oder der inertialen Masse (26) mit einem weichen Dämpfungsmaterial (120) beschichtet oder aus einem Dämpfungsmaterial realisiert sind.

6. Das Modul nach Anspruch 1, wobei:
- der piezoelektrische Wandler zwei koplanare piezoelektrische Längsorgane (22, 22') umfasst, die nebeneinander angeordnet sind, wobei jedes der piezoelektrischen Längsorgane einen so genannten äußeren Schenkel (60) und einen so genannten inneren Schenkel (70) umfasst,
- die proximalen Enden der inneren Schenkel (70) der zwei piezoelektrischen Längsorgane gemeinsam die inertiale Masse (26) stützen und
- die proximalen Enden der äußeren Schenkel (60) der zwei piezoelektrischen Längsorgane mit einem jeweiligen Einspannelement (26) der ringförmigen Montagevorrichtung (100) verbunden sind, wobei die zwei Einspannelemente sich auf beiden Seiten der inertialen Masse (26) in dem Hohlraum befinden.

7. Das Modul nach Anspruch 1, wobei die Position des Schwerpunkts (G) der inertialen Masse (26) im Inneren des Hohlraums der ringförmigen Montagevorrichtung (100) liegt.

8. Das Modul nach Anspruch 1, wobei, gemäß einer axialen Richtung des Längsorgans in seiner größten Abmessung, die Position des Schwerpunkts (G) der inertialen Masse (26) in einer relativ zu der Mitte des Hohlraums der ringförmigen Montagevorrichtung (100) proximalen Richtung versetzt ist.

9. Das Modul nach Anspruch 1, wobei das piezoelektrische Längsorgan an seinem freien distalen Ende ein dazwischen angeordnetes Pendelgewicht (130; 130') an der Stelle der Verbindung (80) zwischen dem äußeren Schenkel (60) und dem inneren Schenkel (70) trägt.

10. Das Modul nach Anspruch 9, wobei das Modul ferner, in der Nähe des distalen Endes des piezoelektrischen Längsorgans, einen Hubbegrenzer (132) umfasst, der imstande ist, einen Anschlag für das dazwischen angeordnete Pendelgewicht (130; 130') in einer maximalen Biegekonfiguration des piezoelektrischen Längsorgans zu bilden.

11. Eine autonome Vorrichtung, die in einem Vorrichtungskörper Folgendes aufnimmt:
- eine elektronische Anordnung (28-38);
- ein Energierückgewinnungsmodul (40) nach einem der vorhergehenden Ansprüche, das ausgangsseitig ein elektrisches Schwingungssignal erzeugt;
- eine Versorgungsverwaltungsschaltung (42), die imstande ist, das von dem Energierückgewinnungmodul erzeugte elektrische Schwingungssignal gleichzurichten und zu regeln, um ausgangsseitig eine stabilisierte Vorsorgungsgleichspannung oder einen stabilisierten Versorgungsgleichstrom bereitzustellen; und
- ein Energiespeicherorgan (44) für die Versorgung der elektronischen Anordnung,
wobei die stabilisierte Gleichspannung oder der stabilisierte Gleichstrom, die/der von der Versorgungsverwaltungsschaltung bereitgestellt werden, der Versorgung der elektronischen Anordnung und/oder der Aufladung des Energiespeicherorgans der autonomen Vorrichtung dienen.

12. Die autonome Vorrichtung nach Anspruch 11, wobei die autonome Vorrichtung eine aktive medizinische Vorrichtung des Typs implantierbare autonome Kapsel (10) ist, die einen Kapselkörper (12) mit einem Element (16) zur Verankerung an einer Wand eines Organs eines Patienten umfasst, und wobei die äußeren Belastungen, die die pendelartige Anordnung (22, 26) des Energierückgewinnungsmoduls erfährt, Belastungen sind, die auf den Kapselkörper (12) unter der Einwirkung von Bewegungen der Wand und/oder von Veränderungen einer Blutflussrate im umgebenden Medium aufgebracht werden.

## Claims

1. An energy harvesting module, comprising a pendular unit subjected to external stresses applied to the module,
the pendular unit comprising a piezoelectric transducer (22) extending, along a central axis (Δ) corresponding to a direction of greater length of the piezoelectric transducer, from a distal end to an opposite proximal end, the transducer being elastically deformable in bending between a clamped end (24) and a free end coupled to an inertial mass (26),
the piezoelectric transducer being adapted to convert a mechanical energy produced by oscillations of the pendular unit into an oscillating electrical signal (V_{OUT}(t)) collected by surface electrodes of the piezoelectric transducer,
wherein:
- the piezoelectric transducer comprises at least one piezoelectric beam (22) configured into two adjacent arms formed single-piece, with an external arm (60) and an internal arm (70) arranged side-by-side,
the external arm (60) having a proximal end (62) fastened to a clamp (24) and a free distal end 64), and the internal arm (70) having a free proximal end (72) supporting the inertial mass (26), and a free distal end (74) connected to the distal end (64) of the external arm (60),
- the module further comprises, around the central axis (Δ), an annular mount (100) that surrounds the piezoelectric beam at its proximal end and that comprises said clamp (24) to which is fastened the proximal end (62) of the external arm (60), and
- the annular mount(100) includes, in a central region in the vicinity of the clamp, a cavity (106) inside which the inertial mass (26) carried by the free proximal end (72) of the internal arm (70) can oscillate transversely to the central axis (Δ).

2. The module of claim 1, wherein the annular mount (100) comprises a stroke limiter having inside the cavity stroke-limiting surfaces (108) adapted to form a stop for the inertial mass (26) in a configuration of maximum bending of the piezoelectric beam.

3. The module of claim 2, wherein the inertial mass (26) comprises stroke-limiting surfaces (114) complementary of the facing stroke-limiting surfaces (108) of the annular mount (100).

4. The module of claim 3, wherein the mutual contact between the stroke-limiting surfaces (108) of the annular mount (100) and the stroke-limiting surfaces (114) of the inertial mass (26) is one of: a plane contact, a linear contact, or a surface-cylindrical contact.

5. The module of claim 2, wherein the stroke-limiting surfaces (108 ; 114) of the annular mount (100) and/or of the inertial mass (26) are coated with a flexible damping material (120), or made of a damping material.

6. The module of claim 1, wherein:
- the piezoelectric transducer comprises two coplanar piezoelectric beams (22, 22') arranged side-by-side, each of the piezoelectric beams comprising a said external arm (60) and a said internal arm (70),
- the proximal ends of the internal arms (70) of the two piezoelectric beams support together the inertial mass (26), and
- the proximal ends of the external arms (60) of the two piezoelectric beams are each connected to a respective clamp (26) of the annular mount (100), the two clamps being located on either side of the inertial mass (26) in the cavity.

7. The module of claim 1, wherein the position of the center of gravity (G) of the inertial mass (26) is located inside the cavity of the annular mount (100).

8. The module of claim 1, wherein, in an axial direction of the beam in its longer dimension, the position of the center of gravity (G) of the inertial mass (26) is offset in a proximal direction with respect to the center of the cavity of the annular mount (100).

9. The module of claim 1, wherein the piezoelectric beam carries at its free distal end an intermediate flyweight (130 ; 130') at the junction (80) between the external arm (60) and the internal arm (70).

10. The module of claim 9, wherein the module further comprises, in the vicinity of the distal end of the piezoelectric beam, a stroke limiter (132) adapted to form a stop for the intermediate flyweight (130; 130') in a configuration of maximum bending of the piezoelectric beam.

11. An autonomous device, housing within a device body:
- an electronic unit (28-38);
- an energy harvesting module (40) according to any one of the previous claims, outputting an oscillating electric signal;
- a power management circuit (42), adapted to rectify and regulate the oscillating electric signal produced by the energy harvesting module, to output stabilized direct voltage or current; and
- an energy storage component (44) for powering the electronic unit,
wherein said stabilized direct voltage or current provided by the power management circuit is used to power the electronic unit and/or to charge the energy storage component of the autonomous device.

12. The autonomous device of claim 11,
wherein the autonomous device is an active medical device of the implantable autonomous capsule (10) type, comprising a capsule body (12) with an element (16) for its anchoring to a wall of a patient's organ,
and wherein the external stresses to which is subjected the pendular unit (22, 26) of the energy harvesting module are stresses applied to the capsule body (12) under the effect of movements of said wall and/or blood flow rate variations in a surrounding environment.
